# EUROPEAN PATENT SPECIFICATION

(11) **EP 0 498 463 B1**
(45) Date of publication and mention of the grant of the patent: **27.09.1995**
(21) Application number: 92102104.4
(22) Date of filing: 07.02.1992
(51) Int. Cl.: A23G 3/00, A23G 3/30, A61K 7/16

(54) **Release-sustained composition for the application to the oral cavity**
Zusammensetzung mit verzögerter Freisetzung für die Anwendung in der Mundhöhle
Composition à délivrance retardée pour l'application à la cavité orale

(30) Priority: 07.02.1991 JP 102202/91
(43) Date of publication of application: 12.08.1992
(73) Proprietor: Ajinomoto Co., Inc., Tokyo 104 (JP)
(72) Inventor: Suzuki, Soji, Cen.Res.Lab.Ajinomoto Co.,Inc., Kawasaki-shi, Kanagawa-ken (US); Harada, Tsutomu, Cen.Res.Lab.Ajinomoto Co.,Inc., Kawasaki-shi, Kanagawa-ken (US)
(74) Representative: Strehl Schübel-Hopf Groening & Partner

(56) References cited:
- EP-A- 0 055 109
- EP-A- 0 059 535
- EP-A- 0 377 278
- DATABASE WPIL Derwent Publications Ltd.,London, GB; DATABASE WPIL, accession no. 86-036833, week 06; & JP-A-61018779 (HOFFMANN LA ROCHE AG) 27.01.1986; & EP-A-170019
- Drogisten Lexikon, H.Irion, Springer Verlag Berlin, Göttingen, Heidelberg (1955), p.610

## Description

The present invention relates to a composition for the application to the oral cavity comprising β-2,1 linked fructose polymer together with an active component, selected from a sweetener component, a component for preventing the development of bad odor in the oral cavity and/or a flavor component and/or a dental anti-caries component.

In prior compositions such as a candy, a chewing gum etc. used for application to the oral cavity, which comprise a component for preventing the development of bad odor in the oral cavity and/or a flavor component and/or a dental anti-caries component, only sugars such as sucrose, glucose, sorbitol and the like have been used as bulking agents for these compositions, although it depends on foods in which the fructose polymers are used. At the same time, sweetness has been imparted by the sugars mentioned above.

When these sugars are used as bulking agents of effective components, however, the active components, whose effects are basically exhibited when they are retained in the oral cavity for a long time dissolve rapidly and are swallowed with sugars so that it is difficult to retain the active components over a long period of time in oral cavity. Therefore, the effects cannot be sufficiently exhibited.

On the other hand, fructose polymer is known to have a physiologically functional property as mentioned in Japanese Published Unexamined Patent Application No. 61-18779. It is thus expected to apply fructose polymer to health food diets, etc. However, fructose polymer has not been effectively applied to any food based on its physical properties and functional properties.

An object of the present invention is to provide a novel composition applicable to the oral cavity which can compensate for the aforesaid defects of compositions for application to the oral cavity, and can prolong the residence time of active components in the oral cavity, whereby the effects of a component for preventing bad odor from the oral cavity and/or a flavor component and/or a dental anti-caries component can be effectively exhibited.

In order to solve the problem, the present inventors have made extensive investigations based on such a hypothesis that if a polysaccharide having a sustained release property and having agreeability to the palate can be incorporated into the composition for application to the oral cavity such as candy and a chewing gum, the residence time in the oral cavity can be prolonged and controlled. As a result, it has been found that the problem can be solved by the following formulation. The problem has thus come to be solved.

It has been found that when β-2,1 linked fructose polymer is incorporated as bulking agents together with one or more active component(s), such as a sweetener component, a component for preventing bad odor development from the oral cavity and/or a flavor component and/or a dental anti-caries component, the residence time of the effective components described above in the oral cavity can be prolonged compared to a conventional composition for application to oral cavity whereby the effects can be enhanced and the residence time can be controlled, that is, the release of these active components can be sustained. The present invention has thus been accomplished.

Upon practice of the present invention, where a dental anti-caries component is contained, it is better not to use sugars such as glucose, sucrose, because they cause the dental caries. It is therefore preferred to use a mixture of a sweetener having a high degree of sweetness which does not cause dental caries, for example, α-L-aspartyl-L-phenylalanine methyl ester (hereafter simply referred to as Aspartame), stevioside, glycyrrhizine, Saccharine, Acesulfam K, or a mixture thereof, and β-2,1 linked fructose polymers as bulking agents.

When a sweetener having a high degree of sweetness is used as the sweetener component, it is sufficient to add the sweetener generally in an amount corresponding to the same sweetness as that of sugars such as sucrose to be replaced, or add the bulking agents composition corresponding to the same sweetness degree as that of sugars such as sucrose to be replaced. Basically, the amount of the sweetener composition used is determined depending upon the size of the composition for oral cavity application, and the amount of sweetener component to be replaced,such as sucrose.

Next the component for preventing the release of bad odor from the oral cavity which is used in the present invention comprises one or more medical plants such as rosemary or sage which have a deodorant effect on bad odorous components like methylmercaptan; seaweeds selected from Eisenia bicyclis, Hijikia fusiformis, Sargussum fulvellum, Chondrus ocellatus, Gracilaria confervoides, Nemacystus and Undaria pinnatifida and mace which also exhibit an effect of preventing periodontal disease, as well as solvent extracts of these materials, purified products of these extracts and/or living organisms and/or dried products thereof. The amount of these materials used is in the range of 0.001 to 50 wt% based on the final product.

As the flavor component which can be used in the present invention, there may be used any flavors which are conventionally used in compositions for application such as a chewing gum to the oral cavity, which are retained in the oral cavity to exhibit a favorable flavor, for example, a flavor of citruses, coffee, tea, mint, herbs.

Next, the anti-dental caries component which can be used in the present invention may be dextranase or mutanase which is an enzyme for decomposing dental plague. As the dextranase, there may be used dextranase obtained by known methods from known dextranase-producing bacteria belonging to Ketosium sp., Penicillium sp., Aspergillus sp., Spicaria sp., Lactobacillus sp., Cellubivrio sp.. This enzyme decomposes α-1,6-glucan which is a kind of dental plague and is incorporated generally in an amount of 100 to 100,000 units per g of the product, when calculated as enzyme.

As the mutanase, there may be used mutanase obtained by known methods from known mutanase-producing bacteria belonging to the Pseudomonas sp., Trichoderma sp., Cladosporium sp., Flavobacterium sp., Streptomyces sp., etc. This enzyme decomposes α-1,3-glucan which is a kind of dental plague and is incorporated generally in an amount of 50 to 10,000 units per g of the product, when calculated as enzyme.

Next, the β-2,1 linked fructose polymer of the present invention refers to a fructose polymer having a β-2,1 bond, for example, inulin, fructose polymer produced by Aspergillus sydowi , etc. or a mixture thereof. Its molecular weight distribution is in the range of 2,000 to 50,000,000, preferably 10,000 to 15,000,000. The amount added is in the range of 1 to 50 wt%, preferably 2 to 30%, based on the final product. With less than 1 wt%, a sufficient effect is not exhibited. Further when the amount exceeds 50%, it is not the case that the polymer may not be used but one would taste a somewhat hard texture though it depends on individual preference of texture. Such a high amount gives an oral cavity composition which might have no agreeability to the palate of consumers. In preparing these oral cavity compositions, the fructose polymer may be used in a powdery form or may be suspended in water, and the suspension and/or solution in water may be used. The form of the fructose polymer may be chosen depending upon its preparation.

The present inventors have studied the control of residence time in the oral cavity as described above by polysaccharides, namely, the effect of imparting sustained release, using a variety of polysaccharides including the fructose polymer. Where it is attempted to impart similar effects, however, polydextrose, gums such as guar gum, other polysaccharides such as starch could not be used because these substances are very hygroscopic, are very slow in dispersion in water, are extremely high in viscosity, are insoluble in water, are gelatinised, or cause an undesired taste with astringency, and a different flavor.

However, it is revealed that the β-2,1 linked fructose polymer used in the present invention has characteristic properties such as a low hygroscopic property, provides an extremely low viscosity when dispersed in water, is gradually dispersed but is free of marked swelling and volume change when dispersed, is quite free of an undesired taste and flavor at all, shows no paste-like texture inherent to other polysaccharides but gives an extremely smooth dispersion, as compared to the aforesaid polysaccharides. That is, it is revealed that the physical properties and textural preference are equivalant to conventional products using sugars, when the fructose β-2,1 linked polymer is used in the oral cavity composition.

Surprisingly, it is also revealed that when the fructose polymer having such properties is incorporated into the oral cavity composition, for example, a candy, malt syrup, a cake tablet, a troche, in preparation thereof, it is possible to control the solubility of these products, especially dissolution velocity alone, by increasing or decreasing the amount of the fructose polymer added. That is, the residence time of the oral cavity composition can be controlled to a desired time period depending upon the amount of addition of β-2,1 linked fructose polymer. Furthermore, it is not observed that the readily soluble component in the oral cavity composition dissolves out predominantly. Therefore, it is realized that the component for preventing bad odor from the oral cavity and/or flavor component and/or anti-dental caries component in accordance with the present invention can be retained for a long time period sufficient to exhibit the function of the effective components satisfactorily, which was impossible in the prior art. That is, these components can be retained in the oral cavity in such a state that the components are imparted by sustained release.

Therefore, the oral cavity composition using the β-2,1 linked fructose polymer does not adversely affect the quality, namely, physical properties such as hygroscopic property and organoleptic preference. The oral cavity composition not only gives preference equivalent to conventional products using sugars but also provides oral cavity compositions such as a candy, malt syrup, a cake tablet, a troche which were not provided in the prior art. The oral cavity composition is not limited to those shown above but also applicable to a chewing gum, a throat troche, a drop candy, a tooth paste, gargle for deodorization, a drug for stomatitis.

### [Effects of the Invention]

According to the present invention, there is provided the oral cavity composition, for example, a candy, malt syrup, a cake tablet, a troche, etc., which is characterized by containing the β-2,1 linked fructose polymer together with a sweetener component, a component for preventing bad odor from the oral cavity and/or a flavor component and/or a dental anti-caries component, whereby the effects of the effective components described above can be enhanced.

Next, the present invention is specifically described by referring to the examples below.

### Example 1

A cake tablet having a coffee flavor was prepared in the following formulation by way of experiment.

For control, glucose was used. In Test groups, fructose polymer produced by Aspergillus sydowi was used in amounts of 50% (Test Group 1) and 25% (Test Group 2), in place of glucose.

| | | |
|---|---|---|
| Control Group: | Glucose | 92.7 % |
| | Aspartame | 0.6 |
| | Instant coffee powder | 0.6 |
| | Coffee flavor | 0.2 |
| | Sodium aspartate | 0.06 |

The raw materials were blended, kneaded and then sieved through a sieve of 50 mesh followed by molding 2.0 g at 60 atms for 2 minutes. Where the fructose polymer described above was substituted by 50%, 46.4% of glucose and 46.3% of fructose polymer were used. In the cases of 25% substitution, 69.5% of glucose and 23.2% fructose polymer were used.

### Example 2

The solubility in Control Group, Test Group 1 and Test Group 2 prepared by way of experiment was examined in terms of dissolution velocity in distilled water at 40°C as a model of the oral cavity.

| | |
|---|---|
| Control Group: | 1.5 minutes |
| Test Group 1: | 150 minutes |
| Test Group 2: | 12.5 minutes |

From the foregoing results, the residence time of the test groups in the oral cavity is prolonged to more than 10 times. In addition, the solubility can be controlled by changing the amount of the fructose polymer formulated, whereby the coffee flavor was gradually released.

### Example 3

In addition to Control Group, Test Group 1 and Test Group 2 prepared by way of experiment above, Test Group 3 was prepared by way of experiment wherein polydextrose was substituted for 50% of glucose. With respect to four groups in total, hygroscopic property under relative humidity of 58% was examined in terms of weight increase by adsorption of humidity and change in appearance.
- Control Group:: hygroscopic rate (increase of weight) 0,5%; no change in appearance
- Test Group 1:: hygroscopic rate 1.9%; no change in appearance
- Test Group 2:: hygroscopic rate 1.2%; no change in appearance
- Test Group 3:: hygroscopic rate 4.9%; deliquescent on the surface
From the foregoing results, it is considered that there is no difference in hygroscopic property between the test groups using the β-2,1 linked fructose polymer and the control group using glucose.

### Example 4

Next, Test Group 1 and Test Group 2 were subjected to organoleptic evaluation (panel persons: 10) and compared to Control Group. The evaluation was made in terms of (1) dissolution time in the oral cavity, (2) quality of taste and (3) preference. (the number of persons who chose it as the best one)
- Test Group 1:: (1) 10 minutes; (2) no difference from the control group; (3) preference: 6
- Test Group 2:: (1) 6.5 minutes; (2) no difference from the control group; (3) preference: 8
- Control Group:: (1) 3.5 minutes; (2) -; (3) preference: 5

As described above, where the fructose polymer was used, a table cake having a coffee flavor with sustained release could be prepared without changing organoleptic or sensory preference.

## Claims

1. A deodorant and/or an anticaries composition for the treatment of the oral cavity comprising β-2,1 linked fructose polymer together with a component for preventing bad odour from the oral cavity and/or a dental anti-caries component.

2. A deodorant and/or an anticaries composition according to Claim 1 additionally comprising a flavour component.

3. A deodorant and/or an anticaries composition according to Claim 1 or Claim 2 wherein further a sweetener is present.

4. A deodorant and/or an anticaries composition according to Claim 3, wherein the sweetener has a high degree of sweetness.

5. A deodorant and/or an anticaries composition according to Claim 3 or Claim 4, wherein α-L-aspartyl-L-phenylalanine methyl ester is present as said sweetener.

6. A deodorant and/or an anticaries composition according to any of the preceding Claims, wherein the molecular weight of the β-2,1 linked fructose polymer is in the range of 2,000 to 50,000,000.

## Patentansprüche

1. Deodorant- und/oder Antikaries-Zusammensetzung zur Behandlung der Mundhöhle, enthaltend ein Fructosepolymer mit β-2,1-Verknüpfung zusammen mit einem Bestandteil zur Verhütung von schlechtem Geruch aus der Mundhöhle und/oder einer dentalen Antikaries-Komponente.

2. Deodorant- und/oder Antikaries-Zusammensetzung nach Anspruch 1, die zusätzlich eine Aromakomponente enthält.

3. Deodorant- und/oder Antikaries-Zusammensetzung nach Anspruch 1 oder Anspruch 2, in der zusätzlich ein Süßstoff vorhanden ist.

4. Deodorant- und/oder Antikaries-Zusammensetzung nach Anspruch 3, wobei der Süßstoff einen hohen Süßgrad hat.

5. Deodorant- und/oder Antikaries-Zusammensetzung nach Anspruch 3 oder Anspruch 4, in der α-L-Aspartyl-L-phenylalaninmethylester als Süßstoff vorliegt.

6. Deodorant- und/oder Antikaries-Zusammensetzung nach einem der vorhergehenden Patentansprüche, wobei das Molekulargewicht des Fructosepolymers mit β-2,1-Verknüpfung im Bereich von 2000 bis 50 000 000 liegt.

## Revendications

1. Composition désodorisante et/ou anti-carie destinée au traitement de la cavité buccale, comprenant du polymère de fructose à liaison β-2,1 avec un composant destiné à empêcher une mauvaise odeur provenant de la cavité buccale et/ou un composant anti-carie dentaire.

2. Composition désodorisante et/ou anti-carie selon la revendication 1 comprenant de plus un composant aromatisant.

3. Composition désodorisante et/ou anti-carie selon la revendication 1 ou la revendication 2, dans laquelle un édulcorant est de plus présent.

4. Composition désodorisante et/ou anti-carie selon la revendication 3, dans laquelle l'édulcorant a un degré élevé de pouvoir sucrant.

5. Composition désodorisante et/ou anti-carie selon la revendication 3 ou la revendication 4, dans laquelle l'ester méthylique d'α-L-aspartyl-L-phénylalanine est présent en tant que ledit édulcorant.

6. Composition désodorisante et/ou anti-carie selon l'une quelconque des revendications précédentes, dans laquelle la masse moléculaire du polymère de fructose à liaison β-2,1 est comprise dans la gamme de 2000 à 50 000 000.
